# EUROPEAN PATENT SPECIFICATION

(11) **EP 3 211 416 B1**
(45) Date of publication and mention of the grant of the patent: **12.09.2018**
(21) Application number: 16157013.0
(22) Date of filing: 23.02.2016
(51) Int. Cl.: G01N 33/00, F24F 11/00

(54) **A BATTERY-POWERED, WIRELESS GAS SENSING UNIT**
BATTERIEBETRIEBENE, DRAHTLOSE GASMESSEINHEIT
UNITÉ DE DÉTECTION DE GAZ SANS FIL ALIMENTÉE PAR UNE BATTERIE

(43) Date of publication of application: 30.08.2017
(73) Proprietor: iQunet BVBA, 9220 Hamme (BE)
(72) Inventor: Vereecken, Wim, 3360 Opvelp (BE); Van den Branden, Dirk, 9220 Hamme (BE)
(74) Representative: IP HILLS NV

(56) References cited:
- WO-A1-2009/011593
- WO-A1-2012/150516
- US-A1- 2003 071 629
- US-A1- 2006 267 756
- JULIA PETTINE ET AL: "Power-Efficient Oscillator-Based Readout Circuit for Multichannel Resonant Volatile Sensors", IEEE TRANSACTIONS ON BIOMEDICAL CIRCUITS AND SYSTEMS, IEEE, US, vol. 6, no. 6, 1 December 2012 (2012-12-01), pages 542-551, XP011487607, ISSN: 1932-4545, DOI: 10.1109/TBCAS.2012.2230629
- KOPPINEN P J ET AL: "A novel MEMS gas sensor based on ultrasonic resonance cavity", 2014 IEEE INTERNATIONAL ULTRASONICS SYMPOSIUM, IEEE, 3 September 2014 (2014-09-03), pages 655-658, XP032667367, DOI: 10.1109/ULTSYM.2014.0161 [retrieved on 2014-10-20]

## Description

### Field of the Invention

The present invention generally relates to gas sensing and particularly to gas sensing units forming parts of the Internet of Things and adapted to measure gas concentrations in atmospheres.

Indoor air quality, also referred to as IAQ, is a term which refers to the air quality within and around buildings and structures, especially as it relates to the health and comfort of building occupants. IAQ can be affected by constituents of air, such as gases, for example carbon monoxide, carbon dioxide or CO2, radon, volatile organic compounds or VOCs, etc., which can induce adverse health conditions. Monitoring the IAQ in homes and buildings comprises measuring gas concentrations in their atmospheres. For example, high CO2 concentrations can be found near vehicle traffic areas, industry and sources of combustion. Fluctuations in air constituents in these atmospheres may indicate events which may be important to relay to homes and buildings occupants.

The Internet of Things is the network of physical objects, such as devices, vehicles, buildings and other items which are embedded with electronics, software, sensors, and network connectivity, which enables these objects to collect and exchange data. The Internet of Things allows objects to be sensed and controlled remotely across existing network infrastructure. Each thing is uniquely identifiable through its embedded computing system but is able to interoperate within the existing Internet infrastructure. These devices collect useful data with the help of various existing technologies and then autonomously flow the data between other devices.

### Background of the Invention

Most heating, ventilation and air conditioning systems, or HVAC systems, present in homes and buildings re-circulate a significant portion of the indoor air to maintain comfort and reduce energy costs associated with heating or cooling outside air. Current technologies allow measurement of CO2 as an indicator to help ensure ventilation systems are delivering the recommended minimum quantities of outside air to buildings occupants. For example, in indoor air atmospheres such as homes in which people exhale CO2, in breweries, in wineries, in restaurants, in dry ice storage, storage tanks, laboratories, in indoor greenhouses, in industrial factories and plants, the monitoring of a CO2 concentration, of the humidity and of the temperature is critical to ensure the CO2 concentration does not reach potentially dangerous levels for a particular industrial/research process and/or for the buildings occupants.

Most gas sensing units present on the market, and especially CO2 sensors, are bulky and are electrically cabled to power lines. The portability of such gas sensing units is then limited. Additionally, most gas sensing units perform continuous gas concentrations measurements. Their power consumption is then non-negligible. There is therefore a need for miniaturized and autonomous gas sensing units with a minimized power consumption.

Additionally, monitoring the IAQ in an industrial factory or plant may be difficult and/or even dangerous for an operator. For example, it may be dangerous to physically access the surroundings of a machine and/or it may be troublesome to suspend the normal operation of a machine in order to perform the measurement of a gas concentration. Additionally, industrial factories or plants are usually wearisome environments for electronic components, making them subject to corrosion and subjecting them to electromagnetic interferences. There is therefore a need for robust gas sensing units comprising means for remotely communicating with a network infrastructure, such as Internet of Things components, thereby not requiring a manual intervention of an operator in the vicinity of the gas sensing unit.

US2015/0077737A1 describes an environmental detector configured to monitor composition of the air surrounding the detector. The detector comprises a temperature and humidity sensor, and one or more gas sensors detecting the concentration level of gaseous species of interest, such as VOCs and CO2. The body of the environmental detector may house one or more batteries, or may be connected to a power supply. The detector fits into a user's hand and may transmit air compositions measurements to a server off board the environmental detector via a transceiver.

Even though the miniaturization and the portability of the environmental detector described in US2015/0077737A1 is made possible by the use of batteries, US2015/0077737A1 discloses that the environmental detector continuously and simultaneously measures any combination of the temperature, the humidity and for example a CO2 concentration in the environment at discrete time intervals in the range of 0.01 milliseconds. This requires the detector to be continuously powered and is therefore battery intensive, thereby reducing the battery lifetime. Even though US2015/0077737A1 describes that the frequency at which the detector simultaneously collects the above measurements may decrease when a user is not present in an environment, there exists a risk that the batteries will frequently need changing, thereby resulting in frequent costs and requiring frequent interventions to change them.

WO2009/011593 A1 discloses a battery-powered, wireless gas sensing unit for measuring a gas concentration, said gas sensing unit comprising a first sensor, a second sensor, a battery unit, a wireless transmitter, a processor and a controller, wherein said first sensor is adapted to continuously measure a value of a first parameter, said second sensor comprises a Volatile Organic Compound sensor, said second sensor and said processor are controlled to measure said gas concentration only when said value of said first parameter or variation of said value of said first parameter exceeds a predefined first parameter threshold, and said wireless transmitter is controlled to transmit said gas concentration only when said gas concentration exceeds a predefined concentration threshold. US2003/071629 A1 discloses a battery-powered gas sensing unit comprising a first sensor, which continuously measures a concentration of a class of chemicals in the atmosphere, and a second sensor, e.g. for carbon dioxide, which is only activated if the concentration measured by the first sensor rises above a threshold. WO2012/150516 A1 discloses a battery-powered, wireless gas sensing unit comprising a first sensor, which periodically measures the concentration of VOCs, and a second sensor for hydrocarbons, which is only activated when the concentration measured by the first sensor exceeds a threshold. US2006/267756 A1 discloses a battery-powered, wireless gas sensing unit, comprising a sensor, which periodically measures a gas concentration and only transmits the signal if the concentration exceeds a threshold, thereby conserving energy.

Low power micro-electro-mechanical systems for detecting volatile organic compounds are known from documents JULIA PETTINE ET AL: "Power-Efficient Oscillator-Based Readout Circuit for Multichannel Resonant Volatile Sensors", IEEE TRANSACTIONS ON BIOMEDICAL CIRCUITS AND SYSTEMS, IEEE, US, vol. 6, no. 6, 1 December 2012 (2012-12-01), pages 542-551, ISSN: 1932-4545, DOI: 10.1109/TBCAS.2012.2230629 and KOPPINEN P J ET AL: "A novel MEMS gas sensor based on ultrasonic resonance cavity", 2014 IEEE INTERNATIONAL ULTRASONICS SYMPOSIUM, IEEE, 3 September 2014 (2014-09-03), pages 655-658, DOI: 10.1109/ULTSYM.2014.0161.

It is an objective of the present invention to disclose a gas sensing unit that overcomes the above identified shortcomings of existing solutions. More particularly, it is an objective to disclose a gas sensing unit demonstrating portability, robustness, intelligence and autonomy.

### Summary of the Invention

According to a first aspect of the present invention, the above defined objectives are realized by a battery-powered, wireless gas sensing unit for measuring a gas concentration, the gas sensing unit comprising a first sensor, a second sensor, a battery unit, a wireless transmitter, a processor and a controller, wherein:
- the first sensor is adapted to periodically measure a value of a first parameter;
- the second sensor comprises a Volatile Organic Compound sensor and said Volatile Organic Compound sensor is a microelectromechanical system;
- the second sensor and the processor are configured to measure the gas concentration only when the value of the first parameter or variation of the value of the first parameter exceeds a predefined first parameter threshold; and
- the wireless transmitter is configured to transmit the gas concentration only when the gas concentration exceeds a predefined concentration threshold.

This way, the lifetime of the battery unit of the gas sensing unit in accordance with the present invention is maximized. The measurement of a value of a first parameter is performed with a low usage of the battery unit of the gas sensing unit. In other words, the measurement of a value of the first parameter is low demanding for the battery unit. A value of the first parameter can then be frequently measured without draining the battery unit of the gas sensing unit. For example, a value of the first parameter may be measured every minute, every 5 minutes, every 10 minutes, every half hour, every hour, every day, etc.. The value of the first parameter is then compared to a predefined first parameter threshold. Alternatively, a variation of the value of the first parameter, for example between two consecutively measured values of the first parameter, or between two measured values of the first parameter, is compared to a predefined first parameter threshold. A measurement of a gas concentration is energy demanding for the battery unit of the gas sensing unit. Therefore, the second sensor is controlled to only measure a gas concentration when the value of the first parameter exceeds the predefined first parameter threshold, or alternatively, only when the variation of the value of the first parameter exceeds the predefined first parameter threshold. In other words, the frequency at which the gas concentration is measured by the second sensor depends on the comparison of the value or of the variation of the value of the first parameter with the predefined first parameter threshold. The gas sensing unit therefore does not perform a continuous and battery intensive measurement of a gas concentration, thereby saving the battery. The value of the gas concentration is then compared to a predefined concentration threshold. The wireless transmitter is controlled to wirelessly transmit the measured gas concentration only when the gas concentration exceeds a predefined concentration threshold, thereby further reducing the impact of the transmission on the battery unit by only transmitting gas concentrations which are indicative for a relevant modification of an atmosphere in the vicinity of the gas sensing unit. In other words, the battery consumption of the gas sensing unit according to the present invention is minimized thanks to the use of a first sensor which frequently measures a value of a first parameter with low impact on the battery life and of a second sensor which only measures the gas concentration when triggered by the measurement of the first sensor and of a transmitter which is controlled to only transmit the gas concentration when required.

Additionally, the design of the gas sensing unit according to the present invention is made compact by the integration of the first sensor, the second sensor, the battery unit, the wireless transmitted, the processor and the controller in a single gas sensing unit. The battery unit ensures the energy independence of the gas sensing unit, which does not require to be electrically cabled to power lines. Additionally, the gas sensing unit is encapsulated in a housing which shelters the components of the gas sensing unit, thereby rendering the gas sensing unit according to the present invention more robust against corrosion and electromagnetic interferences. Additionally, the gas sensing unit may be remotely updated when performing an update remotely received by a wireless transceiver comprising the wireless transmitter.

In accordance with the present invention, the first parameter threshold and the predefined concentration threshold may be programmed. Exceeding the first parameter threshold or exceeding the predefined concentration threshold corresponds to respectively a value of the first parameter being equal to or larger than the first parameter threshold, or to respectively a gas concentration being equal to or larger than the predefined concentration threshold. Alternatively, exceeding the first parameter threshold or exceeding the predefined concentration threshold corresponds to respectively a value of the first parameter being smaller than or equal to the first parameter threshold, or to respectively a gas concentration being smaller than or equal to the predefined concentration threshold.

According to an optional aspect of the invention, the first sensor comprises one or more of the group of:
- a temperature sensor;
- a humidity sensor;
- a presence sensor, configured to detect presence of a living entity in the vicinity of the battery-powered, wireless gas sensing unit;
- a proximity sensor, configured to detect presence of a living entity or an object within a predefined distance from the battery-powered, wireless gas sensing unit;
- a movement sensor, configured to detect movement of a living entity or an object in the vicinity of the battery-powered, wireless gas sensing unit.

This way, the first parameter is the temperature of an atmosphere in the vicinity of the gas sensing unit. Alternatively, the first parameter is the humidity of an atmosphere in the vicinity of the gas sensing unit. Measuring a value of the temperature or a value of the humidity has a low impact on the battery unit of the gas sensing unit. Therefore, the power consumption required for frequently measuring a value of the first parameter is minimized. Moreover, a change in temperature or in humidity that exceeds a certain threshold may be indicative for a change in constitution of the atmosphere. Alternatively, the first parameter is presence in the vicinity of the gas sensing unit. For example, the first parameter is presence of a living entity, such as of a human being, of an animal, etc. Presence in the vicinity of the gas sensing unit may result in a change in constitution of the atmosphere. For example, presence of one or more human beings may result in an increase in the CO2 concentration of the atmosphere. Alternatively, the first parameter is proximity to the gas sensing unit. For example, proximity is presence of a machine, of an object, of a living entity, etc. within a predefined distance from the gas sensing unit. Alternatively, the first parameter is detection of a movement in the vicinity of the gas sensing unit. For example, a movement of a machine, of an object, of a living entity, etc.

The periodic monitoring of values of the first parameter increases the reactivity of the gas sensing unit to fluctuations of the first parameter. The period of the measurements of values of the first parameter may be programmed, and may be modified. Additionally, periodically measuring values of the first parameter allows building an indexed data collection which can be used to compare a measured value to previously measured values. For example, a value of a first parameter measured at a given time on a given day may be compared to the value of the first parameter which was measured at the same time the day before, or at the same time two days before, or at the same time a week before, or at the same time on the same day at which a similar event occurred in the vicinity of the gas sensing unit.

According to an optional aspect of the invention, the first sensor is adapted to measure the value of the first parameter with a periodicity comprised between once per second and once per hour.

This way, the measurements of values of the first parameter have a low impact on the lifetime of the battery unit, while still ensuring that a fluctuation of the first parameter remains detectable in time for the second sensor and the processor to be controlled to measure a gas concentration.

Values of concentrations of volatile organic compounds in an atmosphere in the vicinity of the gas sensing unit can be measured. Volatile organic compounds are organic chemicals that have a high vapor pressure at ordinary room temperature. Their high vapor pressure results from a low boiling point, which causes large numbers of molecules to evaporate or sublimate from the liquid or solid form of the compound and enter the surrounding air. Some VOCs are dangerous to human health or cause harm to the environment. A building occupant could be alerted by another device receiving a VOC concentration transmitted by the wireless transmitter when the VOC concentration exceeds a predefined concentration threshold. VOCs concentrations can only be correctly determined when the second sensor is heated to a temperature suitable for measuring VOC concentrations, for example a temperature comprised between 300°C and 450°C, preferably 400°C.

Microelectromechanical systems or MEMs are being used as Volatile Organic Compound sensors or VOC sensors. MEMs demonstrate small dimensions, for example in the range of millimetre square, which further improves the compactness of the gas sensing unit. Additionally, MEMs have a very low power consumption, for example less than hundreds of milli-Watt, and preferably even less than tens of milli-Watt, thereby further maximizing the lifetime of the battery unit.

According to an optional aspect of the invention, the gas concentration is a CO2 concentration.

This way, the gas sensing unit measures a CO2 concentration to help ensure ventilation and air conditioning systems are delivering the recommended minimum quantities of outside air to buildings occupants. The monitoring of a CO2 concentration is critical in ensuring the CO2 concentration does not reach hazardous levels of CO2 concentration.

According to an optional aspect of the invention, the processor is configured to calculate the CO2 concentration from output of the Volatile Organic Compound sensor.

This way, a bulky CO2 sensor is not required anymore in order to measure a CO2 concentration. The small dimensions of the VOC sensor ensure the compactness of the gas sensing unit, while allowing to determine a CO2 concentration from the output of the VOC sensor.

According to an optional aspect of the invention, the battery unit comprises a first battery and a second battery, and the second battery is parallel connected to the first battery while the wireless transmitter transmits the gas concentration.

This way, the second battery of the battery unit is only used when the wireless transmitter transmits the gas concentration. This maximizes the lifetime of the second battery.

According to an optional aspect of the invention, the gas sensing unit further comprises a calibration unit adapted to supply an electrical current to the second sensor prior to each measurement of the gas concentration, the electrical current being determined to bring the second sensor at a same operational working point.

This way, the second sensor always operates at the same operational working point for all the measurements of gas concentrations. In other words, all the gas concentrations are measured in the same operational conditions of the second sensor. This ensures the comparison of values of the gas concentrations to the predefined concentration threshold stays relevant, and further ensures that the comparison of values of the gas concentrations to previously measured values of the gas concentrations is relevant. This way, a real determination of fluctuations of gas concentrations is made possible. Before every measurement of a gas concentration, the gas sensing unit check if the second sensor operates at the correct operational working point. For example, the calibration unit supplies an electrical current to the second sensor such that the second sensor always operates under the same temperature conditions.

According to an optional aspect of the invention, the gas sensing unit further comprises:
- an Analog-to-Digital Converter, adapted to digitize output values of the second sensor;
- a high impedance current source, having an output coupled to the second sensor and being configured to supply a current to the second sensor such that output values of the second sensor stay within a limited central interval of the operational range of the Analog-to-Digital Converter; and
- the processor is configured to consider the current when calculating the gas concentration from the output values of the second sensor.

This way, a current is imposed during the measurements of the second sensors to ensure the measurements of the gas concentrations are performed within a limited central interval of the operational range of the Analog-to-Digital Converter or ADC. The value of this imposed current is then used by the processor when calculating the gas concentrations from the output values of the second sensor. This way, an ADC with a small voltage operational range can be chosen to be integrated in the gas sensing unit. This further minimizes the costs associated with the implementation of the gas sensing unit.

According to an optional aspect of the invention, the gas sensing unit further comprises an encrypting module, adapted to perform encryption prior to transmission.

This way, prior to transmission, the gas concentrations are encoded in order to only allow authorized parties to read them and/or modify them when and after they have been transmitted.

According to an optional aspect of the invention, the gas sensing further comprises an error correction unit, adapted to perform error correction prior to transmission.

This way, prior to transmission, the gas concentrations are encoded and the gas sensing unit performs error correction techniques to control errors in data transmission over unreliable or noisy communication channels and to minimize the risk of the presence of an error. For example, Forward Error Correction or FEC, Read Solomon Coding or RS, turbocoding, Hamming Oriented Error Correction are suitable error correction techniques for the gas sensing unit.

According to a second aspect of the invention, there is provided a method comprising the steps of:
- periodically measuring a value of a first parameter with a first sensor;
- providing a second sensor comprising a Volatile Organic Compound sensor, wherein the Volatile Organic Compound sensor is a microelectromechanical system;
- measuring with the second sensor the gas concentration only when the value of the first parameter or variation of the value of the first parameter exceeds a predefined first parameter threshold; and
- wirelessly transmitting the gas concentration only when the gas concentration exceeds a predefined concentration threshold.

This way, the lifetime of a battery unit of a gas sensing unit performing the steps of the method in accordance with the present invention is maximized. The measurement of a value of a first parameter is performed with a low usage of the battery unit of the gas sensing unit. In other words, the measurement of a value of the first parameter is low demanding for the battery unit. A value of the first parameter can then be frequently measured without draining the battery unit of the gas sensing unit. For example, a value of the first parameter may be measured every minute, every 5 minutes, every 10 minutes, every half hour, every hour, every day, etc.. The value of the first parameter is then compared to a predefined first parameter threshold. Alternatively, a variation of the value of the first parameter, for example between two consecutively measured values of the first parameter, or between two measured values of the first parameter, is compared to a predefined first parameter threshold. A measurement of a gas concentration is energy demanding for the battery unit of the gas sensing unit. Therefore, a gas concentration is only measured when the value of the first parameter exceeds the predefined first parameter threshold, or alternatively, only when the variation of the value of the first parameter exceeds the predefined first parameter threshold. In other words, the frequency at which the gas concentration is measured depends on the comparison of the value or of the variation of the value of the first parameter with the predefined first parameter threshold. The gas concentrations are not continuously measured, thereby saving the battery. The value of the gas concentration is then compared to a predefined concentration threshold. The measured gas concentration is only transmitted when the gas concentration exceeds a predefined concentration threshold, thereby further reducing the impact of the transmission by only transmitting gas concentrations which are indicative for a relevant modification of an atmosphere in the vicinity of the gas sensing unit. In other words, the battery consumption of a gas sensing unit performing the steps of the method in accordance with the present invention is minimized thanks to the frequent measurement of a value of a first parameter with low impact on the battery life and of the controlled measurement of a gas concentration and of a transmission of a gas concentration only when required.

In accordance with the present invention, the first parameter threshold and the predefined concentration threshold may be programmed. Exceeding the first parameter threshold or exceeding the predefined concentration threshold corresponds to respectively a value of the first parameter being larger than or equal to the first parameter threshold, or to respectively a gas concentration being larger than or equal to the predefined concentration threshold. Alternatively, exceeding the first parameter threshold or exceeding the predefined concentration threshold corresponds to respectively a value of the first parameter being smaller than or equal to the first parameter threshold, or to respectively a gas concentration being smaller than or equal to the predefined concentration threshold.

### Brief Description of the Drawings

Fig. 1 schematically illustrates an embodiment of a battery-powered, wireless gas sensing unit according to the present invention.
Fig. 2 schematically illustrates an embodiment of a battery-powered, wireless gas sensing unit, wherein a first sensor comprises a temperature sensor and a humidity sensor.
Fig. 3 schematically illustrates an embodiment of a battery-powered, wireless gas sensing unit, wherein a second sensor comprises a VOC sensor from which a CO2 concentration is calculated.
Fig. 4 schematically illustrates an embodiment of a battery-powered, wireless gas sensing unit, wherein a battery unit comprises a first battery and a second battery.
Fig. 5 schematically illustrates an embodiment of a battery-powered, wireless gas sensing unit comprising a calibration unit.
Fig. 6 schematically illustrates an embodiment of a battery-powered, wireless gas sensing unit comprising an Analog-to-Digital Converter and a high impedance current source.
Fig. 7 schematically illustrates a front view of an example of a housing suitable for hosting a battery-powered, wireless gas sensing unit according to the present invention.

### Detailed Description of Embodiment(s)

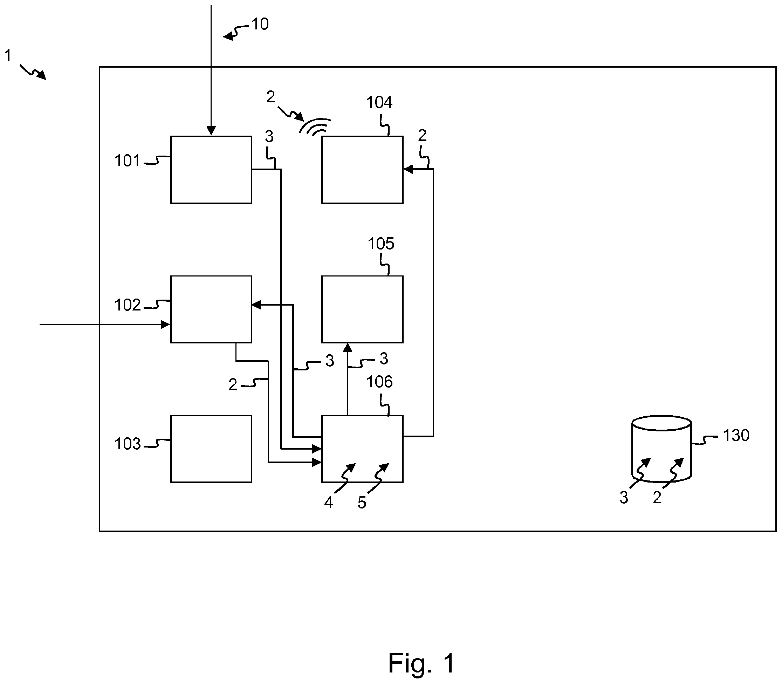

According to an embodiment shown in Fig. 1, a battery-operated, wireless gas sensing unit 1 comprises a first sensor 101, a second sensor 102, a battery unit 103, a wireless transmitter 104, a processor 105, a controller 106 and a memory 130. Crossing lines in Fig. 1 are not to be interpreted as connections. The first sensor 101 periodically measures a value 3 of a first parameter 10. The first sensor 101 can for example measure a value 3 of a first parameter 10 every 30 seconds, every minute, every 2 minutes, every 5 minutes, every 10 minutes, every 30 minutes, every hour, every 12 hours, or every 24 hours, etc.. According to an alternative embodiment, the first sensor 101 periodically measures a value 3 of a first parameter 10. For example, the first sensor 101 measures a value 3 of a first parameter 10 several times per minute, or several times per hour, or several times per day, etc. A value 3 of a first parameter 10 may be stored in the memory 130. The controller 106 compares the value 3 of the first parameter 10 measured by the first sensor 101 to a predefined first parameter threshold 4. The predefined first parameter threshold 4 may be programmed in the controller 106. According to an alternative embodiment, the predefined first parameter threshold 4 is remotely received by a wireless transceiver comprising the wireless transmitter 104 and sent to the controller 106. According to an alternative embodiment, the predefined first parameter threshold 4 is retrieved from the memory 130. The value of the predefined first parameter threshold 4 may be modified. A value 3 of the first parameter 10 exceeds the predefined first parameter threshold 4 when the value 3 is equal to or larger than the first parameter threshold 4. According to an alternative embodiment, a value 3 of the first parameter 10 exceeds the predefined first parameter threshold 4 when the value 3 is smaller than or equal to the first parameter threshold 4. Only when a value 3 of the first parameter 10 exceeds the predefined first parameter threshold 4 does the controller 106 controls the second sensor 102 and the processor 105 to measure a gas concentration 2. A gas concentration 2 may be a carbon monoxide concentration or a Volatile Organic Compounds concentration. A gas concentration 2 may be stored in the memory 130. The controller 106 compares the gas concentration 2 measured by the second sensor 102 to a predefined concentration threshold 5. The predefined concentration threshold 5 may be programmed in the controller 106. According to an alternative embodiment, the predefined concentration threshold 5 is remotely received by the wireless transmitter 104 and sent to the controller 106. According to an alternative embodiment, the predefined concentration threshold 5 is retrieved from the memory 130. The value of the predefined concentration threshold 5 may be modified. A gas concentration 2 exceeds the predefined concentration threshold 5 when the gas concentration 2 is equal to or larger than the predefined concentration threshold 5. According to an alternative embodiment, a gas concentration 2 exceeds the predefined concentration threshold 5 when the gas concentration 2 is smaller than or equal to the predefined concentration threshold 5. Only when a gas concentration 2 exceeds the predefined concentration threshold 5 does the controller 106 control the wireless transmitter 104 to transmit the gas concentration 2.

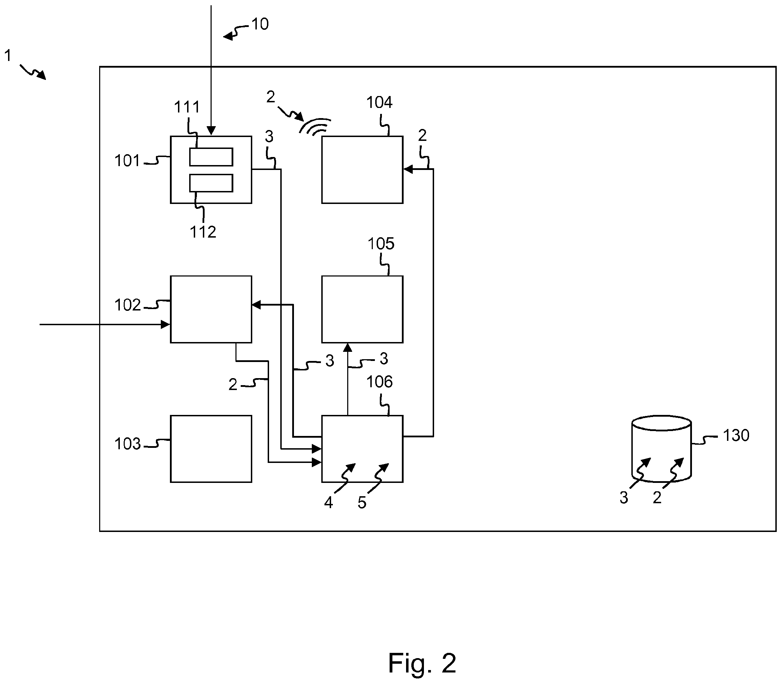

According to an embodiment shown in Fig. 2, a battery-operated, wireless gas sensing unit 1 comprises a first sensor 101, a second sensor 102, a battery unit 103, a wireless transmitter 104, a processor 105, a controller 106 and a memory 130. Crossing lines in Fig. 2 are not to be interpreted as connections. Components having identical reference numbers to components in Fig. 1 perform the same function. The first sensor 101 comprises a temperature sensor 111 and a humidity sensor 112. According to an alternative embodiment, the first sensor 101 comprises a temperature sensor 111 or a humidity sensor 112. According to an alternative embodiment, the first sensor 101 comprises one or more of the group of a temperature sensor 111, a humidity sensor 112, a presence sensor, a proximity sensor, a movement sensor. A value 3 of the first parameter 10 measured by the first sensor 101 is a value 3 of the temperature of the environment of the gas sensing unit 1. According to an alternative embodiment, a value 3 of the first parameter 10 measured by the first sensor 101 is a value 3 of the humidity of the environment of the gas sensing unit 1. According to an alternative embodiment, the first parameter 10 measured by the first sensor 101 is any combination of the values 3 of the temperature and the humidity of the environment of the gas sensing unit 1. The first sensor 101 periodically measures a value 3 of a first parameter 1. The first sensor 101 can for example measure a value 3 of a first parameter 10 every 30 seconds, every minute, every 2 minutes, every 5 minutes, every 10 minutes, every 30 minutes, every hour, every 12 hours, or every 24 hours, etc.. According to an alternative embodiment, the first sensor 101 periodically measures a value 3 of a first parameter 10. For example, the first sensor 101 measures a value 3 of a first parameter 10 several times per minute, or several times per hour, or several times per day, etc. A value 3 of a first parameter 10 may be stored in the memory 130. The controller 106 compares the value 3 of the first parameter 10 measured by the first sensor 101 to a predefined first parameter threshold 4. The predefined first parameter threshold 4 may be programmed in the controller 106. According to an alternative embodiment, the predefined first parameter threshold 4 is remotely received by a wireless transceiver comprising the wireless transmitter 104 and sent to the controller 106. According to an alternative embodiment, the predefined first parameter threshold 4 is retrieved from the memory 130. The value of the predefined first parameter threshold 4 may be modified. A value 3 of the first parameter 10 exceeds the predefined first parameter threshold 4 when the value 3 is equal to or larger than the first parameter threshold 4. According to an alternative embodiment, a value 3 of the first parameter 10 exceeds the predefined first parameter threshold 4 when the value 3 is smaller than or equal to the first parameter threshold 4. Only when a value 3 of the first parameter 10 exceeds the predefined first parameter threshold 4 does the controller 106 controls the second sensor 102 and the processor 105 to measure a gas concentration 2. A gas concentration 2 may be a carbon monoxide concentration or a Volatile Organic Compounds concentration. A gas concentration 2 may be stored in the memory 130. The controller 106 compares the gas concentration 2 measured by the second sensor 102 to a predefined concentration threshold 5. The predefined concentration threshold 5 may be programmed in the controller 106. According to an alternative embodiment, the predefined concentration threshold 5 is remotely received by the wireless transmitter 104 and sent to the controller 106. According to an alternative embodiment, the predefined concentration threshold 5 is retrieved from the memory 130. The value of the predefined concentration threshold 5 may be modified. A gas concentration 2 exceeds the predefined concentration threshold 5 when the gas concentration 2 is equal to or larger than the predefined concentration threshold 5. According to an alternative embodiment, a gas concentration 2 exceeds the predefined concentration threshold 5 when the gas concentration 2 is smaller than or equal to the predefined concentration threshold 5. Only when a gas concentration 2 exceeds the predefined concentration threshold 5 does the controller 106 controls the wireless transmitter 104 to transmit the gas concentration 2.

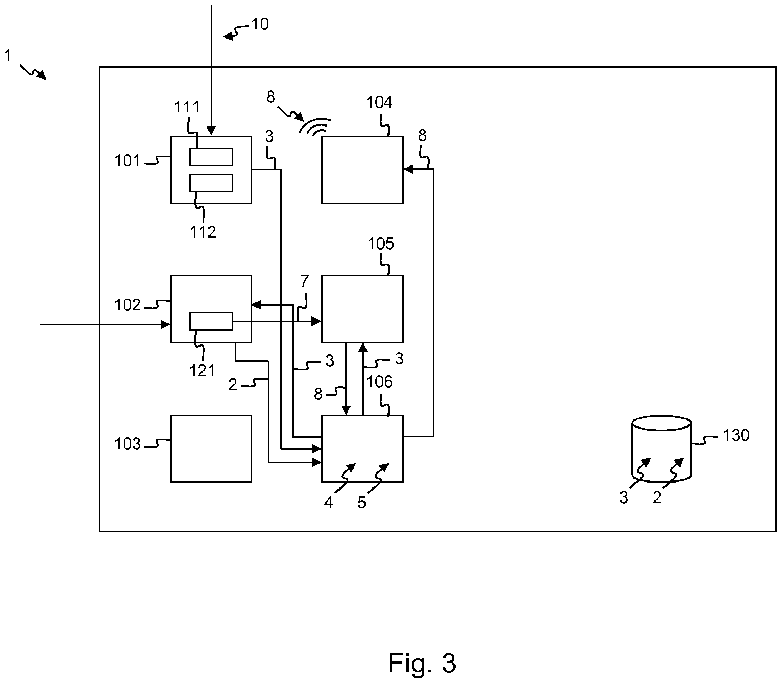

According to an embodiment shown in Fig. 3, a battery-operated, wireless gas sensing unit 1 comprises a first sensor 101, a second sensor 102, a battery unit 103, a wireless transmitter 104, a processor 105, a controller 106 and a memory 130. Crossing lines in Fig. 3 are not to be interpreted as connections. Components having identical reference numbers to components in Fig. 2 perform the same function. The first sensor 101 comprises a temperature sensor 111 and a humidity sensor 112. According to an alternative embodiment, the first sensor 101 comprises a temperature sensor 111 or a humidity sensor 112. According to an alternative embodiment, the first sensor 101 comprises one or more of the group of a temperature sensor 111, a humidity sensor 112, a presence sensor, a proximity sensor, a movement sensor. A value 3 of the first parameter 10 measured by the first sensor 101 is a value 3 of the temperature of the environment of the gas sensing unit 1. According to an alternative embodiment, a value 3 of the first parameter 10 measured by the first sensor 101 is a value 3 of the humidity of the environment of the gas sensing unit 1. According to an alternative embodiment, the first parameter 10 measured by the first sensor 101 is any combination of the values 3 of the temperature and the humidity of the environment of the gas sensing unit 1. The first sensor 101 periodically measures a value 3 of a first parameter 10. The first sensor 101 can for example measure a value 3 of a first parameter 10 every 30 seconds, every minute, every 2 minutes, every 5 minutes, every 10 minutes, every 30 minutes, every hour, every 12 hours, or every 24 hours, etc.. According to an alternative embodiment, the first sensor 101 periodically measures a value 3 of a first parameter 10. For example, the first sensor 101 measures a value 3 of a first parameter 10 several times per minute, or several times per hour, or several times per day, etc. A value 3 of a first parameter 10 may be stored in the memory 130. The controller 106 compares the value 3 of the first parameter 10 measured by the first sensor 101 to a predefined first parameter threshold 4. The predefined first parameter threshold 4 may be programmed in the controller 106. According to an alternative embodiment, the predefined first parameter threshold 4 is remotely received by a wireless transceiver comprising the wireless transmitter 104 and sent to the controller 106. According to an alternative embodiment, the predefined first parameter threshold 4 is retrieved from the memory 130. The value of the predefined first parameter threshold 4 may be modified. A value 3 of the first parameter 10 exceeds the predefined first parameter threshold 4 when the value 3 is equal to or larger than the first parameter threshold 4. According to an alternative embodiment, a value 3 of the first parameter 10 exceeds the predefined first parameter threshold 4 when the value 3 is smaller than or equal to the first parameter threshold 4. Only when a value 3 of the first parameter 10 exceeds the predefined first parameter threshold 4 does the controller 106 controls the second sensor 102 and the processor 105 to measure a gas concentration 2. The second sensor 102 comprises a Volatile Organic Compound sensor 121 or VOC sensor 121. The VOC sensor 121 is a microelectromechanical system or MEM. The dimensions of the MEM are for example 2x3 millimetres, or a few millimetres square. The MEMs have a low power consumption, for example less than hundreds of milli-Watt, and preferably even less than tens of milli-Watt. The VOC sensor 121 is heated up to a temperature between 350°C and 450°C, preferably to 400°C. A gas concentration 2 may be a carbon monoxide concentration or a Volatile Organic Compounds concentration. A gas concentration 2 may be stored in the memory 130. The processor 105 calculates a CO2 concentration 8 from output 7 of the VOC sensor 121. The controller 106 compares the CO2 concentration 8 calculated from output of the VOC sensor 121 to a predefined concentration threshold 5. The controller 106 may also compare a gas concentration 2 measured by the second sensor 102 to a predefined concentration threshold 5. The predefined concentration threshold 5 may be programmed in the controller 106. According to an alternative embodiment, the predefined concentration threshold 5 is remotely received by the wireless transmitter 104 and sent to the controller 106. According to an alternative embodiment, the predefined concentration threshold 5 is retrieved from the memory 130. The value of the predefined concentration threshold 5 may be modified. A CO2 concentration 8 exceeds the predefined concentration threshold 5 when the CO2 concentration 8 is equal to or larger than the predefined concentration threshold 5. According to an alternative embodiment, a CO2 concentration 8 exceeds the predefined concentration threshold 5 when the CO2 concentration 8 is smaller than or equal to the predefined concentration threshold 5. Only when a CO2 concentration 8 exceeds the predefined concentration threshold 5 does the controller 106 controls the wireless transmitter 104 to transmit the CO2 concentration 8.

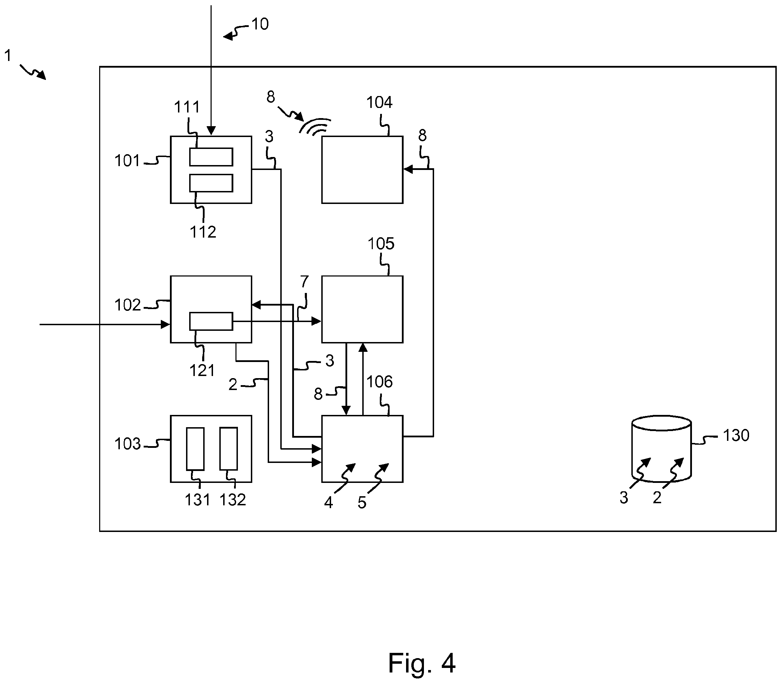

According to an embodiment shown in Fig. 4, a battery-operated, wireless gas sensing unit 1 comprises a first sensor 101, a second sensor 102, a battery unit 103, a wireless transmitter 104, a processor 105, a controller 106 and a memory 130. Crossing lines in Fig. 4 are not to be interpreted as connections. Components having identical reference numbers to components in Fig. 3 perform the same function. The first sensor 101 comprises a temperature sensor 111 and a humidity sensor 112. According to an alternative embodiment, the first sensor 101 comprises a temperature sensor 111 or a humidity sensor 112. According to an alternative embodiment, the first sensor 101 comprises one or more of the group of a temperature sensor 111, a humidity sensor 112, a presence sensor, a proximity sensor, a movement sensor. A value 3 of the first parameter 10 measured by the first sensor 101 is a value 3 of the temperature of the environment of the gas sensing unit 1. According to an alternative embodiment, a value 3 of the first parameter 10 measured by the first sensor 101 is a value 3 of the humidity of the environment of the gas sensing unit 1. According to an alternative embodiment, the first parameter 10 measured by the first sensor 101 is any combination of the values 3 of the temperature and the humidity of the environment of the gas sensing unit 1. The first sensor 101 periodically measures a value 3 of a first parameter 10. For example, the first sensor 101 measures a value 3 of a first parameter 10 periodically. The first sensor 101 can for example measure a value 3 of a first parameter 10 every 30 seconds, every minute, every 2 minutes, every 5 minutes, every 10 minutes, every 30 minutes, every hour, every 12 hours, or every 24 hours, etc.. According to an alternative embodiment, the first sensor 101 periodically measures a value 3 of a first parameter 10. For example, the first sensor 101 measures a value 3 of a first parameter 10 several times per minute, or several times per hour, or several times per day, etc. A value 3 of a first parameter 10 may be stored in the memory 130. The controller 106 compares the value 3 of the first parameter 10 measured by the first sensor 101 to a predefined first parameter threshold 4. The predefined first parameter threshold 4 may be programmed in the controller 106. According to an alternative embodiment, the predefined first parameter threshold 4 is remotely received by a wireless transceiver comprising the wireless transmitter 104 and sent to the controller 106. According to an alternative embodiment, the predefined first parameter threshold 4 is retrieved from the memory 130. The value of the predefined first parameter threshold 4 may be modified. A value 3 of the first parameter 10 exceeds the predefined first parameter threshold 4 when the value 3 is equal to or larger than the first parameter threshold 4. According to an alternative embodiment, a value 3 of the first parameter 10 exceeds the predefined first parameter threshold 4 when the value 3 is smaller than or equal to the first parameter threshold 4. Only when a value 3 of the first parameter 10 exceeds the predefined first parameter threshold 4 does the controller 106 controls the second sensor 102 and the processor 105 to measure a gas concentration 2. The second sensor 102 comprises a Volatile Organic Compound sensor 121 or VOC sensor 121. A gas concentration 2 may for example be a CO2 concentration 8. According to alternative embodiments, a gas concentration 2 may be a carbon monoxide concentration or a Volatile Organic Compound concentration. A gas concentration 2 may be stored in the memory 130. The processor 105 calculates a CO2 concentration 8 from output 7 of the VOC sensor 121. The controller 106 compares the CO2 concentration 8 calculated from output of the VOC sensor 121 to a predefined concentration threshold 5. The controller 106 may also compare a gas concentration 2 measured by the second sensor 102 to a predefined concentration threshold 5. The predefined concentration threshold 5 may be programmed in the controller 106. According to an alternative embodiment, the predefined concentration threshold 5 is remotely received by the wireless transmitter 104 and sent to the controller 106. According to an alternative embodiment, the predefined concentration threshold 5 is retrieved from the memory 130. The value of the predefined concentration threshold 5 may be modified. A CO2 concentration 8 exceeds the predefined concentration threshold 5 when the CO2 concentration 8 is equal to or larger than the predefined concentration threshold 5. According to an alternative embodiment, a CO2 concentration 8 exceeds the predefined concentration threshold 5 when the CO2 concentration 8 is smaller than or equal to the predefined concentration threshold 5. Only when a CO2 concentration 8 exceeds the predefined concentration threshold 5 does the controller 106 controls the wireless transmitter 104 to transmit the CO2 concentration 8. The battery unit 103 comprises a first battery 131 and a second battery 132. The second battery 132 is parallel connected to the first battery 131 while the wireless transmitter 104 transmits the CO2 concentration 8. According to an alternative embodiment, the second battery 132 is parallel connected to the first battery 131 while the wireless transmitter 104 transmits the gas concentration 2.

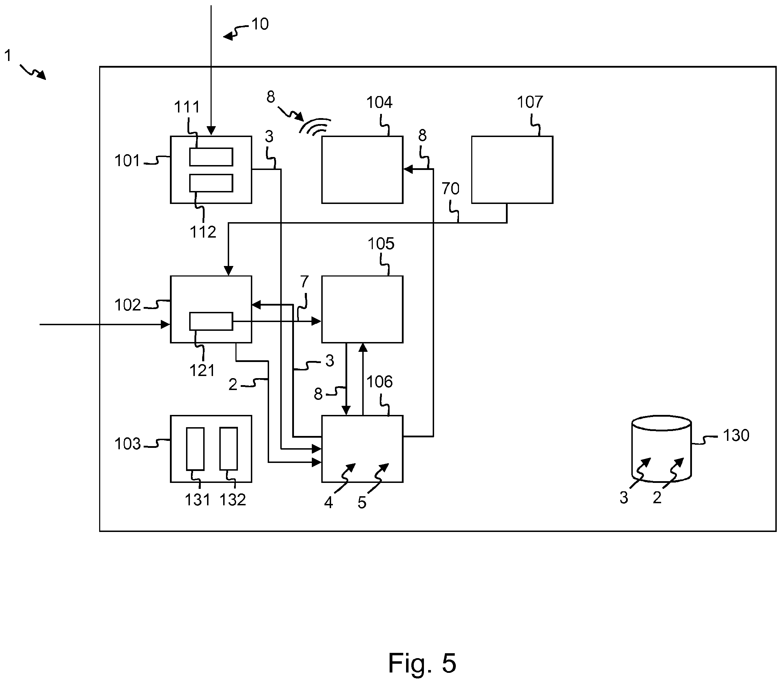

According to an embodiment shown in Fig. 5, a battery-operated, wireless gas sensing unit 1 comprises a first sensor 101, a second sensor 102, a battery unit 103, a wireless transmitter 104, a processor 105, a controller 106, a memory 130 and a calibration unit 107. Crossing lines in Fig. 5 are not to be interpreted as connections. Components having identical reference numbers to components in Fig. 4 perform the same function. The first sensor 101 comprises a temperature sensor 111 and a humidity sensor 112. According to an alternative embodiment, the first sensor 101 comprises a temperature sensor 111 or a humidity sensor 112. According to an alternative embodiment, the first sensor 101 comprises one or more of the group of a temperature sensor 111, a humidity sensor 112, a presence sensor, a proximity sensor, a movement sensor. A value 3 of the first parameter 10 measured by the first sensor 101 is a value 3 of the temperature of the environment of the gas sensing unit 1. According to an alternative embodiment, a value 3 of the first parameter 10 measured by the first sensor 101 is a value 3 of the humidity of the environment of the gas sensing unit 1. According to an alternative embodiment, the first parameter 10 measured by the first sensor 101 is any combination of the values 3 of the temperature and the humidity of the environment of the gas sensing unit 1. The first sensor 101 periodically measures a value 3 of a first parameter 10. The first sensor 101 can for example measure a value 3 of a first parameter 10 every 30 seconds, every minute, every 2 minutes, every 5 minutes, every 10 minutes, every 30 minutes, every hour, every 12 hours, or every 24 hours, etc.. According to an alternative embodiment, the first sensor 101 periodically measures a value 3 of a first parameter 10. For example, the first sensor 101 measures a value 3 of a first parameter 10 several times per minute, or several times per hour, or several times per day, etc. A value 3 of a first parameter 10 may be stored in the memory 130. The controller 106 compares the value 3 of the first parameter 10 measured by the first sensor 101 to a predefined first parameter threshold 4. The predefined first parameter threshold 4 may be programmed in the controller 106. According to an alternative embodiment, the predefined first parameter threshold 4 is remotely received by a wireless transceiver comprising the wireless transmitter 104 and sent to the controller 106. According to an alternative embodiment, the predefined first parameter threshold 4 is retrieved from the memory 130. The value of the predefined first parameter threshold 4 may be modified. A value 3 of the first parameter 10 exceeds the predefined first parameter threshold 4 when the value 3 is equal to or larger than the first parameter threshold 4. According to an alternative embodiment, a value 3 of the first parameter 10 exceeds the predefined first parameter threshold 4 when the value 3 is smaller than or equal to the first parameter threshold 4. Only when a value 3 of the first parameter 10 exceeds the predefined first parameter threshold 4 does the controller 106 controls the second sensor 102 and the processor 105 to measure a gas concentration 2. The second sensor 102 comprises a Volatile Organic Compound sensor 121 or VOC sensor 121. A gas concentration 2 may for example be a CO2 concentration 8. According to alternative embodiments, a gas concentration 2 may be a carbon monoxide or CO concentration or a Volatile Organic Compound concentration. A gas concentration 2 may be stored in the memory 130. The processor 105 calculates a CO2 concentration 8 from output 7 of the VOC sensor 121. The controller 106 compares the CO2 concentration 8 calculated from output of the VOC sensor 121 to a predefined concentration threshold 5. The controller 106 may also compare a gas concentration 2 measured by the second sensor 102 to a predefined concentration threshold 5. The predefined concentration threshold 5 may be programmed in the controller 106. According to an alternative embodiment, the predefined concentration threshold 5 is remotely received by the wireless transmitter 104 and sent to the controller 106. According to an alternative embodiment, the predefined concentration threshold 5 is retrieved from the memory 130. The value of the predefined concentration threshold 5 may be modified. A CO2 concentration 8 exceeds the predefined concentration threshold 5 when the CO2 concentration 8 is equal to or larger than the predefined concentration threshold 5. According to an alternative embodiment, a CO2 concentration 8 exceeds the predefined concentration threshold 5 when the CO2 concentration 8 is smaller than or equal to the predefined concentration threshold 5. Only when a CO2 concentration 8 exceeds the predefined concentration threshold 5 does the controller 106 controls the wireless transmitter 104 to transmit the CO2 concentration 8. The battery unit 103 comprises a first battery 131 and a second battery 132. The second battery 132 is parallel connected to the first battery 131 while the wireless transmitter 104 transmits the CO2 concentration 8. According to an alternative embodiment, the second battery 132 is parallel connected to the first battery 131 while the wireless transmitter 104 transmits the gas concentration 2. Prior to each measurement of a gas concentration 2 by the second sensor 102, the calibration unit 107 supplies an electrical current 70 to the second sensor 102. According to an alternative embodiment, the calibration unit 107 supplies an electrical current 70 to the VOC sensor 121 prior to each measurement of a gas concentration 2 by the second sensor 102. The electrical current 70 is determined to bring the second sensor 102 at the same operational point. The electrical current 70 may be determined by the controller 106. According to an alternative embodiment, the electrical current 70 may be determined by the processor 105. According to an alternative embodiment, the electrical current 70 may be remotely programmed.

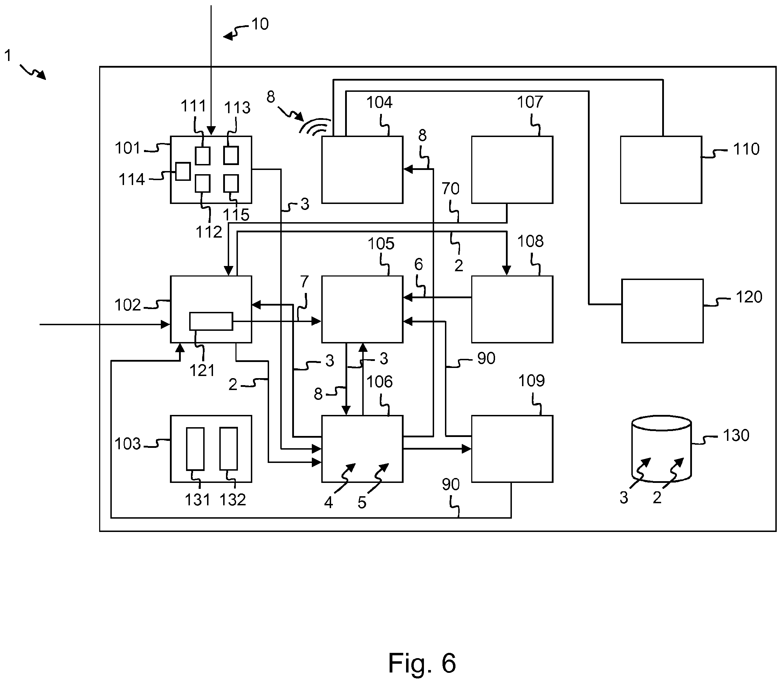

According to an embodiment shown in Fig. 6, a battery-operated, wireless gas sensing unit 1 comprises a first sensor 101, a second sensor 102, a battery unit 103, a wireless transmitter 104, a processor 105, a controller 106, a memory 130, a calibration unit 107, an Analog-to-Digital Converter or ADC 108, a high impedance current source 109, an encryption module 110 and an error correction unit 120. Crossing lines in Fig. 6 are not to be interpreted as connections. Components having identical reference numbers to components in Fig. 5 perform the same function. The first sensor 101 comprises a temperature sensor 111 and a humidity sensor 112. According to an alternative embodiment, the first sensor 101 comprises a temperature sensor 111 or a humidity sensor 112. According to an alternative embodiment, the first sensor 101 comprises one or more of the group of a temperature sensor 111, a humidity sensor 112, a presence sensor 113, a proximity sensor 114, a movement sensor 115. A value 3 of the first parameter 10 measured by the first sensor 101 is a value 3 of the temperature of the environment of the gas sensing unit 1. According to an alternative embodiment, a value 3 of the first parameter 10 measured by the first sensor 101 is a value 3 of the humidity of the environment of the gas sensing unit 1. According to an alternative embodiment, the first parameter 10 measured by the first sensor 101 is any combination of the values 3 of the temperature and the humidity of the environment of the gas sensing unit 1. The first sensor 101 periodically measures a value 3 of a first parameter 10. The first sensor 101 can for example measure a value 3 of a first parameter 10 every 30 seconds, every minute, every 2 minutes, every 5 minutes, every 10 minutes, every 30 minutes, every hour, every 12 hours, or every 24 hours, etc.. According to an alternative embodiment, the first sensor 101 periodically measures a value 3 of a first parameter 10. For example, the first sensor 101 measures a value 3 of a first parameter 10 several times per minute, or several times per hour, or several times per day, etc. A value 3 of a first parameter 10 may be stored in the memory 130. The controller 106 compares the value 3 of the first parameter 10 measured by the first sensor 101 to a predefined first parameter threshold 4. The predefined first parameter threshold 4 may be programmed in the controller 106. According to an alternative embodiment, the predefined first parameter threshold 4 is remotely received by a wireless transceiver comprising the wireless transmitter 104 and sent to the controller 106. According to an alternative embodiment, the predefined first parameter threshold 4 is retrieved from the memory 130. The value of the predefined first parameter threshold 4 may be modified. A value 3 of the first parameter 10 exceeds the predefined first parameter threshold 4 when the value 3 is equal to or larger than the first parameter threshold 4. According to an alternative embodiment, a value 3 of the first parameter 10 exceeds the predefined first parameter threshold 4 when the value 3 is smaller than or equal to the first parameter threshold 4. Only when a value 3 of the first parameter 10 exceeds the predefined first parameter threshold 4 does the controller 106 controls the second sensor 102 and the processor 105 to measure a gas concentration 2. The second sensor 102 comprises a Volatile Organic Compound sensor 121 or VOC sensor 121. A gas concentration 2 may for example be a CO2 concentration 8. According to alternative embodiments, a gas concentration 2 may be a carbon monoxide concentration or a Volatile Organic Compound concentration. A gas concentration 2 may be stored in the memory 130. The processor 105 calculates a CO2 concentration 8 from output 7 of the VOC sensor 121. The controller 106 compares the CO2 concentration 8 calculated from output of the VOC sensor 121 to a predefined concentration threshold 5. The controller 106 may also compare a gas concentration 2 measured by the second sensor 102 to a predefined concentration threshold 5. The predefined concentration threshold 5 may be programmed in the controller 106. According to an alternative embodiment, the predefined concentration threshold 5 is remotely received by the wireless transmitter 104 and sent to the controller 106. According to an alternative embodiment, the predefined concentration threshold 5 is retrieved from the memory 130. The value of the predefined concentration threshold 5 may be modified. A CO2 concentration 8 exceeds the predefined concentration threshold 5 when the CO2 concentration 8 is equal to or larger than the predefined concentration threshold 5. According to an alternative embodiment, a CO2 concentration 8 exceeds the predefined concentration threshold 5 when the CO2 concentration 8 is smaller than or equal to the predefined concentration threshold 5. Only when a CO2 concentration 8 exceeds the predefined concentration threshold 5 does the controller 106 controls the wireless transmitter 104 to transmit the CO2 concentration 8. The battery unit 103 comprises a first battery 131 and a second battery 132. The second battery 132 is parallel connected to the first battery 131 while the wireless transmitter 104 transmits the CO2 concentration 8. According to an alternative embodiment, the second battery 132 is parallel connected to the first battery 131 while the wireless transmitter 104 transmits the gas concentration 2. Prior to each measurement of a gas concentration 2 by the second sensor 102, the calibration unit 107 supplies an electrical current 70 to the second sensor 102. According to an alternative embodiment, the calibration unit 107 supplies an electrical current 70 to the VOC sensor 121 prior to each measurement of a gas concentration 2 by the second sensor 102. The electrical current 70 is determined to bring the second sensor 102 at the same operational point. The electrical current 70 may be determined by the controller 106. According to an alternative embodiment, the electrical current 70 may be determined by the processor 105. According to an alternative embodiment, the electrical current 70 may be remotely programmed. The ADC 108 digitizes output values 6 of the second sensor 102. The high impedance current source 109 has an output coupled to the second sensor 102. The controller 106 controls the high impedance current source 109 to supply a current 90 to the second sensor 102 such that output values 6 of the second sensor 102 stay within a limited central interval of the operational range of the ADC 108. The processor 105 considers the current 90 when calculating the gas concentration 2 from the output values 6 of the second sensor 102. According to an alternative embodiment, the ADC 108 digitizes output values 6 of the VOC sensor 121. The high impedance current source 109 has an output coupled to the VOC sensor 121. The controller 106 controls the high impedance current source 109 to supply a current 90 to the VOC sensor 121 such that output values 7 of the VOC sensor 121 stay within a limited central interval of the operational range of the ADC 108. The processor 105 considers the current 90 when calculating the CO2 concentration 8 from the output values 7 of the VOC sensor 121. The encryption module 110 performs encryption prior to transmission by the wireless transmitter 104. For example, the encryption module 110 encrypts a gas concentration 2 and/or a CO2 concentration 8 prior to transmission. The error correction unit 120 performs error correction techniques prior to transmission by the wireless transmitter 104. The error correction unit 120 for example performs error correct techniques such as Forward Error Correction or FEC, Read Solomon Coding or RS, turbocoding, Hamming Oriented Error Correction.

The gas sensing unit 1 shown in Fig. 6 is designed such that the leakage currents are minimized, such that for example the leakage currents are as low as 2µA in sleep mode. This further reduces the usage of the battery unit 103. Prior to the measurement of a gas concentration 2, and particularly prior to the measurement of a CO2 concentration 8, a battery level of the first battery 131 is measured in order to determine if the measurement of the gas concentration 2, and particularly the measurement of the CO2 concentration 8, can be performed.

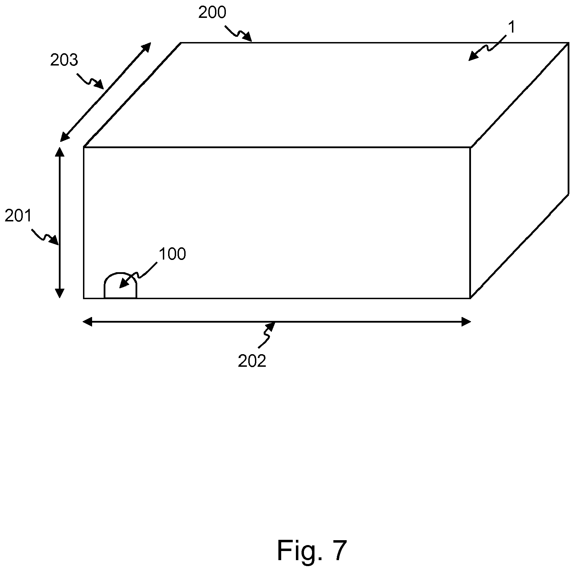

According to an example shown in Fig. 7, a housing 200 is suitable for hosting a battery-powered, wireless gas sensing unit according to any of Fig. 1 to 6. As depicted in Fig. 7, the housing 200 may demonstrate a box-alike shape. According to alternative examples, the housing 200 may be a cube, a sphere, or any or suitable shape. The housing depicted in Fig. 7 for example has a height 201 of 15 millimetres, a length 202 of 50 millimetres and a width 203 of 30 millimetres. The housing 200 comprises an opening 100 adapted to allow an air circulation in the gas sensing unit 1. In other words, the opening 100 allows air to freely flow in the housing 200. The gas sensing unit 1 can then for example measure a temperature, a humidity, a gas concentration, etc. of the air in the environment of the gas sensing unit 1. According to an alternative example, the opening 100 may be positioned on any surface of the housing 200, or on any corner or edge of the housing 200, even overlapping a surface of the housing 100. The opening 100 of the housing 200 depicted in Fig. 7 is half circular. According to alternative examples, the opening 100 is circular, square, triangular, rectangular, or can have any suitable shape. The housing 200 may comprise means for holding on for example a machine, a wall, a ceiling, etc..

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof, as defined by the appended claims. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A battery-powered, wireless gas sensing unit (1) for measuring a gas concentration (2), said gas sensing unit (1) comprising a first sensor (101), a second sensor (102), a battery unit (103), a wireless transmitter (104), a processor (105) and a controller (106), wherein:
- said first sensor (101) is adapted to periodically measure a value (3) of a first parameter (10);
- said second sensor (102) comprises a Volatile Organic Compound sensor (121) and said Volatile Organic Compound sensor (121) is a microelectromechanical system;
- said second sensor (102) and said processor (105) are configured to measure said gas concentration (2) only when said value (3) of said first parameter (10) or variation of said value (3) of said first parameter (10) exceeds a predefined first parameter threshold (4); and
- said wireless transmitter (104) is configured to transmit said gas concentration (2) only when said gas concentration (2) exceeds a predefined concentration threshold (5).

2. A gas sensing unit (1) according to claim 1, wherein said first sensor (101) comprises one or more of the group of:
- a temperature sensor (111);
- a humidity sensor (112);
- a presence sensor (113), configured to detect presence of a living entity in the vicinity of said battery-powered, wireless gas sensing unit (1);
- a proximity sensor (114), configured to detect presence of a living entity or an object within a predefined distance from said battery-powered, wireless gas sensing unit (1);
- a movement sensor (115), configured to detect movement of a living entity or an object in the vicinity of said battery-powered, wireless gas sensing unit (1).

3. A gas sensing unit (1) according to claim 1 or 2, wherein said first sensor (101) is adapted to measure said value (3) of said first parameter (10) with a periodicity comprised between once per second and once per hour.

4. A gas sensing unit (1) according to any of the preceding claims, wherein said gas concentration (2) is a CO₂ concentration (8).

5. A gas sensing unit (1) according to claim 4, wherein said processor (105) is configured to calculate said CO₂ concentration (8) from output (7) of said Volatile Organic Compound sensor (121).

6. A gas sensing unit (1) according to any of the preceding claims, wherein said battery unit (103) comprises a first battery (131) and a second battery (132), and wherein said gas sensing unit (1) is configured such that said second battery (132) is parallel connected to said first battery (131) while said wireless transmitter (104) transmits said gas concentration (2).

7. A gas sensing unit (1) according to any of the preceding claims, wherein said gas sensing unit (1) further comprises a calibration unit (107) adapted to supply an electrical current (70) to said second sensor (102) prior to each measurement of said gas concentration (2), said electrical current (70) being determined to bring said second sensor (102) at a same operational working point.

8. A gas sensing unit (1) according to any of the preceding claims, wherein said gas sensing unit (1) further comprises:
- an Analog-to-Digital Converter (108), adapted to digitize output values (6) of said second sensor (102);
- a high impedance current source (109), having an output coupled to said second sensor (102) and being configured to supply a current (90) to said second sensor (102) such that output values (6) of said second sensor (102) stay within a central interval of the operational range of said Analog-to-Digital Converter (108); and
- said processor (105) is configured to consider said current (90) when calculating said gas concentration (2) from said output values (6) of said second sensor (102).

9. A gas sensing unit (1) according to any of the preceding claims, further comprising an encrypting module (110), adapted to perform encryption prior to transmission.

10. A gas sensing unit (1) according to any of the preceding claims, further comprising an error correction unit (120), adapted to perform error correction prior to transmission.

11. A method for measuring a gas concentration (2), said method comprising the steps of:
- periodically measuring a value (3) of a first parameter (10) with a first sensor (101);
- providing a second sensor (102) comprising a Volatile Organic Compound sensor (121), wherein said Volatile Organic Compound sensor (121) is a microelectromechanical system;
- measuring with said second sensor (102) said gas concentration (2) only when said value (3) of said first parameter (10) or variation of said value (3) of said first parameter (10) exceeds a predefined first parameter threshold (4); and
- wirelessly transmitting said gas concentration (2) only when said gas concentration (2) exceeds a predefined concentration threshold (5).

## Patentansprüche

1. Batteriebetriebene drahtlose Gasmesseinheit (1) zum Messen einer Gaskonzentration (2), wobei die Gasmesseinheit (1) einen ersten Sensor (101), einen zweiten Sensor (102), eine Batterieeinheit (103), einen drahtlosen Sender (104), einen Prozessor (105) und ein Steuergerät (106) umfasst, wobei:
- der erste Sensor (101) angepasst ist, um in regelmäßigen Abständen einen Wert (3) eines ersten Parameters (10) zu messen;
- der zweite Sensor (102) einen Sensor für flüchtige organische Verbindungen (121) umfasst und der Sensor für flüchtige organische Verbindungen (121) ein mikroelektromechanisches System ist;
- der zweite Sensor (102) und der Prozessor (105) konfiguriert sind, um die Gaskonzentration (2) nur zu messen, wenn der Wert (3) des ersten Parameters (10) oder eine Veränderung des Werts (3) des ersten Parameters (10) eine vordefinierte erste Parameterschwelle (4) überschreitet; und
- der drahtlose Sender (104) konfiguriert ist, um die Gaskonzentration (2) nur zu übertragen, wenn die Gaskonzentration (2) einen vordefinierten Konzentrationsschwellenwert (5) übersteigt.

2. Gasmesseinheit (1) nach Anspruch 1, wobei der erste Sensor (101) einen oder mehrere von der folgenden Gruppe umfasst:
- einen Temperatursensor (111);
- einen Feuchtigkeitssensor (112);
- einen Anwesenheitssensor (113), der konfiguriert ist, um die Anwesenheit eines Lebewesens in der Nähe der batteriebetriebenen drahtlosen Gasmesseinheit (1) zu detektieren;
- einen Näherungssensor (114), der konfiguriert ist, um die Anwesenheit eines Lebewesens oder eines Objekts in einem vorbestimmten Abstand von der batteriebetriebenen drahtlosen Gasmesseinheit (1) zu detektieren;
- einen Bewegungssensor (115), der konfiguriert ist, um eine Bewegung eines Lebewesens oder eines Objekts in der Nähe der batteriebetriebenen drahtlosen Gasmesseinheit (1) zu detektieren.

3. Gasmesseinheit (1) nach Anspruch 1 oder 2, wobei der erste Sensor (101) angepasst ist, um den Wert (3) des ersten Parameters (10) mit einer Häufigkeit zu messen, die zwischen einmal pro Sekunde und einmal pro Stunde umfasst.

4. Gasmesseinheit (1) nach einem der vorhergehenden Ansprüche, wobei die Gaskonzentration (2) eine CO₂-Konzentration (8) ist.

5. Gasmesseinheit (1) nach Anspruch 4, wobei der Prozessor (105) konfiguriert ist, um die CO₂-Konzentration (8) aus der Ausgabe (7) des Sensors für flüchtige organische Verbindungen (121) zu berechnen.

6. Gasmesseinheit (1) nach einem der vorhergehenden Ansprüche, wobei die Batterieeinheit (103) eine erste Batterie (131) und eine zweite Batterie (132) umfasst und wobei die Gasmesseinheit (1) konfiguriert ist, sodass die zweite Batterie (132) parallel zu der ersten Batterie (131) geschaltet ist, während der drahtlose Sender (104) die Gaskonzentration (2) überträgt.

7. Gasmesseinheit (1) nach einem der vorhergehenden Ansprüche, wobei die Gasmesseinheit (1) ferner eine Kalibriereinheit (107) umfasst, die angepasst ist, um dem zweiten Sensor (102) vor jeder Messung der Gaskonzentration (2) einen elektrischen Strom (70) zu liefern, wobei der elektrische Strom (70) bestimmt wird, um den zweiten Sensor (102) auf einen gleichen Betriebsarbeitspunkt zu bringen.

8. Gasmesseinheit (1) nach einem der vorhergehenden Ansprüche, wobei die Gasmesseinheit (1) ferner Folgendes umfasst:
- einen Analog-Digital-Umsetzer (108), der angepasst ist, um von dem zweiten Sensor (102) ausgegebene Werte (6) zu digitalisieren;
- eine hochohmige Stromquelle (109), die einen Ausgang aufweist, der an den Sensor (102) gekoppelt ist, und konfiguriert ist, um dem zweiten Sensor (102) einen Strom (90) zu liefern, sodass Ausgabewerte (6) des zweiten Sensors (102) in einem mittleren Intervall des Betriebsbereichs des Analog-Digital-Umsetzers (108) bleiben; und
- der Prozessor (105) konfiguriert ist, um den Strom (90) bei dem Berechnen der Gaskonzentration (2) aus den Ausgabewerten (6) des zweiten Sensors (102) zu berücksichtigen.

9. Gasmesseinheit (1) nach einem der vorhergehenden Ansprüche, die ferner ein Verschlüsselungsmodul (110) umfasst, das angepasst ist, um vor einer Übertragung eine Verschlüsselung durchzuführen.

10. Gasmesseinheit (1) nach einem der vorhergehenden Ansprüche, die ferner eine Fehlerkorrektureinheit (120) umfasst, die angepasst ist, um vor einer Übertragung eine Fehlerkorrektur durchzuführen.

11. Verfahren zum Messen einer Gaskonzentration (2), wobei das Verfahren die folgenden Schritte umfasst:
- periodisches Messen eines Werts (3) eines ersten Parameters (10) mit einem ersten Sensor (101);
- Bereitstellen eines zweiten Sensors (102), der einen Sensor für flüchtige organische Verbindungen (121) umfasst, wobei der Sensor für flüchtige organische Verbindungen (121) ein mikroelektromechanisches System ist;
- Messen der Gaskonzentration (2) mit dem zweiten Sensor (102) nur, wenn der Wert (3) des ersten Parameters (10) oder eine Veränderung des Werts (3) des ersten Parameters (10) einen vordefinierten ersten Parameterschwellenwert (4) überschreitet; und
- drahtloses Übertragen der Gaskonzentration (2) nur, wenn die Gaskonzentration (2) einen vordefinierten Konzentrationsschwellenwert (5) überschreitet.

## Revendications

1. Unité de détection de gaz (1) sans fil alimentée par batterie destinée à mesurer une concentration de gaz (2), ladite unité de détection de gaz (1) comprenant un premier capteur (101), un second capteur (102), une unité de batterie (103), un transmetteur sans fil (104), un processeur (105) et un dispositif de commande (106),
- ledit premier capteur (101) étant conçu pour mesurer périodiquement une valeur (3) d'un premier paramètre (10) ;
- ledit second capteur (102) comprenant un capteur de composé organique volatil (121) et ledit capteur de composé organique volatil (121) étant un système microélectromécanique ;
- ledit second capteur (102) et ledit processeur (105) étant configurés pour mesurer ladite concentration de gaz (2) uniquement lorsque ladite valeur (3) dudit premier paramètre (10) ou d'une variation de ladite valeur (3) dudit premier paramètre (10) dépasse un seuil (4) prédéfini de premier paramètre ; et
- ledit transmetteur sans fil (104) étant configuré pour transmettre ladite concentration de gaz (2) uniquement lorsque ladite concentration de gaz (2) dépasse un seuil (5) prédéfini de concentration.

2. Unité de détection de gaz (1) selon la revendication 1, ledit premier capteur (101) comprenant l'un ou plusieurs du groupe constitué de :
- un capteur de température (111) ;
- un capteur d'humidité (112) ;
- un capteur de présence (113) configuré pour détecter la présence d'une entité vivante dans le voisinage de ladite unité de détection de gaz (1) sans fil alimentée par batterie ;
- un capteur de proximité (114) configuré pour détecter la présence d'une entité vivante ou d'un objet jusqu'à une distance prédéfinie depuis ladite unité de détection de gaz (1) sans fil alimentée par batterie ;
- un capteur de mouvement (115) configuré pour détecter un mouvement d'une entité vivante ou d'un objet dans le voisinage de ladite unité de détection de gaz (1) sans fil alimentée par batterie.

3. Unité de détection de gaz (1) selon la revendication 1 ou 2, ledit premier capteur (101) étant conçu pour mesurer ladite valeur (3) dudit premier paramètre (10) avec une périodicité comprise entre une fois par seconde et une fois par heure.

4. Unité de détection de gaz (1) selon l'une quelconque des revendications précédentes, ladite concentration de gaz (2) étant une concentration de CO₂ (8).

5. Unité de détection de gaz (1) selon la revendication 4, ledit processeur (105) étant configuré pour calculer ladite concentration de CO₂ (8) depuis la sortie (7) dudit capteur de composé organique volatil (121).

6. Unité de détection de gaz (1) selon l'une quelconque des revendications précédentes, ladite unité de batterie (103) comprenant une première batterie (131) et une seconde batterie (132) et ladite unité de détection de gaz (1) étant configurée de sorte que ladite seconde batterie (132) soit connectée en parallèle à ladite première batterie (131) tandis que ledit transmetteur sans fil (104) transmet ladite concentration de gaz (2).

7. Unité de détection de gaz (1) selon l'une quelconque des revendications précédentes, ladite unité de détection de gaz (1) comprenant en outre une unité d'étalonnage (107) conçue pour fournir un courant électrique (70) audit second capteur (102) avant chaque mesure de ladite concentration de gaz (2), ledit courant électrique (70) étant déterminé pour amener ledit second capteur (102) au même point de travail fonctionnel.

8. Unité de détection de gaz (1) selon l'une quelconque des revendications précédentes, ladite unité de détection de gaz (1) comprenant en outre :
- un convertisseur analogique-numérique (108) conçu pour numériser des valeurs de sortie (6) dudit second capteur (102) ;
- une source de courant à impédance élevée (109) possédant une sortie couplée audit second capteur (102) et étant configuré pour fournir un courant (90) audit second capteur (102) de sorte que des valeurs de sortie (6) dudit second capteur (102) restent dans un intervalle central de la plage de fonctionnement dudit convertisseur analogique-numérique (108) ; et
- ledit processeur (105) étant configuré pour considérer ledit courant (90) lors du calcul de ladite concentration de gaz (2) à partir desdites valeurs de sortie (6) dudit second capteur (102).

9. Unité de détection de gaz (1) selon l'une quelconque des revendications précédentes, comprenant en outre un module de chiffrement (110) conçu pour effectuer un chiffrement avant la transmission.

10. Unité de détection de gaz (1) selon l'une quelconque des revendications précédentes, comprenant en outre une unité de correction d'erreur (120) conçue pour effectuer une correction d'erreur avant la transmission.

11. Procédé permettant la mesure d'une concentration de gaz (2) ledit procédé comprenant les étapes de :
- mesure périodique d'une valeur (3) d'un premier paramètre (10) avec un premier capteur (101) ;
- fourniture d'un second capteur (102) comprenant un capteur de composé organique volatil (121), ledit capteur de composé organique volatil (121) étant un système microélectromécanique ;
- mesure avec ledit second capteur (102) de ladite concentration de gaz (2) uniquement lorsque ladite valeur (3) dudit premier paramètre (10) ou la variation de ladite valeur (3) dudit premier paramètre (10) dépasse un seuil prédéfini de premier paramètre (4) ; et
- transmission sans fil de ladite concentration de gaz (2) uniquement lorsque ladite concentration de gaz (2) dépasse un seuil prédéfini de concentration (5).
